# EUROPEAN PATENT APPLICATION

(11) **EP 2 199 385 A1**
(43) Date of publication of application: **23.06.2010**
(21) Application number: 08021836.5
(22) Date of filing: 16.12.2008
(51) Int. Cl.: C12N 5/10, C07K 14/01, C07K 14/075, C12N 5/16

(54) **Specific and persistent activation of heat shock response in cell lines using a viral factor**

(71) Applicant: ProBioGen AG, 13086 Berlin (DE)
(72) Inventor: Jordan, Ingo, Dr., 13156 Berlin (DE); Sandig, Volker, Dr., 13158 Berlin (DE)
(74) Representative: Zwicker, Jörk

(57) **Abstract**

This application relates to cells that permanentely sustain a heat shock cascade without undergoing apoptosis.

SEQUENCE LISTING

<110> ProBioGen AG

<120> Specific and persistent activation of heat shock response in cell
lines using a viral factor

<130> 513-10

<160> 17

<170> PatentIn version 3.5

<210> 1
<211> 140
<212> PRT
<213> Human adenovirus type 5

<400> 1

Met Ser Thr Asn Ser Phe Asp Gly Ser Ile Val Ser Ser Tyr Leu Thr
1               5                   10                  15

Thr Arg Met Pro Pro Trp Ala Gly Val Arg Gln Asn Val Met Gly Ser
            20                  25                  30

Ser Ile Asp Gly Arg Pro Val Leu Pro Ala Asn Ser Thr Thr Leu Thr
        35                  40                  45

Tyr Glu Thr Val Ser Gly Thr Pro Leu Glu Thr Ala Ala Ser Ala Ala
    50                  55                  60

Ala Ser Ala Ala Ala Ala Thr Ala Arg Gly Ile Val Thr Asp Phe Ala
65                  70                  75                  80

Phe Leu Ser Pro Leu Ala Ser Ser Ala Ala Ser Arg Ser Ser Ala Arg
                85                  90                  95

Asp Asp Lys Leu Thr Ala Leu Leu Ala Gln Leu Asp Ser Leu Thr Arg
            100                 105                 110

Glu Leu Asn Val Val Ser Gln Gln Leu Leu Asp Leu Arg Gln Gln Val
        115                 120                 125

Ser Ala Leu Lys Ala Ser Ser Pro Pro Asn Ala Val
    130                 135                 140

<210> 2
<211> 990
<212> DNA
<213> Human adenovirus type 5

<400> 2
atgagacata ttatctgcca cggaggtgtt attaccgaag aaatggccgc cagtcttttg 60

gaccagctga tcgaagaggt actggctgat aatcttccac ctcctagcca ttttgaacca 120

cctacccttc acgaactgta tgatttagac gtgacggccc ccgaagatcc caacgaggag 180

gcggtttcgc agatttttcc cgactctgta atgttggcgg tgcaggaagg gattgactta 240
ctcacttttc cgccggcgcc cggttctccg gagccgcctc acctttcccg gcagcccgag 300
cagccggagc agagagcctt gggtccggtt tctatgccaa accttgtacc ggaggtgatc 360
gatcttacct gccacgaggc tggctttcca cccagtgacg acgaggatga agagggtgag 420
gagtttgtgt tagattatgt ggagcacccc gggcacggtt gcaggtcttg tcattatcac 480
cggaggaata cgggggaccc agatattatg tgttcgcttt gctatatgag gacctgtggc 540
atgtttgtct acagtaagtg aaaattatgg gcagtgggtg atagagtggt gggtttggtg 600
tggtaatttt ttttttaatt tttacagttt tgtggtttaa agaattttgt attgtgattt 660
ttttaaaagg tcctgtgtct gaacctgagc ctgagcccga gccagaaccg gagcctgcaa 720
gacctacccg ccgtcctaaa atggcgcctg ctatcctgag acgcccgaca tcacctgtgt 780
ctagagaatg caatagtagt acggatagct gtgactccgg tccttctaac acacctcctg 840
agatacaccc ggtggtcccg ctgtgcccca ttaaaccagt tgccgtgaga gttggtgggc 900
gtcgccaggc tgtggaatgt atcgaggact tgcttaacga gcctgggcaa cctttggact 960
tgagctgtaa acgccccagg ccataaggtg 990

<210> 3
<211> 1796
<212> DNA
<213> Human adenovirus type 5

<400> 3
atggaggctt gggagtgttt ggaagatttt tctgctgtgc gtaacttgct ggaacagagc 60
tctaacagta cctcttggtt ttggaggttt ctgtggggct catcccaggc aaagttagtc 120
tgcagaatta aggaggatta caagtgggaa tttgaagagc ttttgaaatc ctgtggtgag 180
ctgtttgatt ctttgaatct gggtcaccag gcgcttttcc aagagaaggt catcaagact 240
ttggattttt ccacaccggg gcgcgctgcg gctgctgttg cttttttgag ttttataaag 300
gataaatgga gcgaagaaac ccatctgagc ggggggtacc tgctggattt tctggccatg 360
catctgtgga gagcggttgt gagacacaag aatcgcctgc tactgttgtc ttccgtccgc 420
ccggcgataa taccgacgga ggagcagcag cagcagcagg aggaagccag gcggcggcgg 480
caggagcaga gcccatggaa cccgagagcc ggcctggacc ctcgggaatg aatgttgtac 540
aggtggctga actgtatcca gaactgagac gcattttgac aattacagag gatgggcagg 600
ggctaaaggg ggtaaagagg gagcgggggg cttgtgaggc tacagaggag gctaggaatc 660
tagcttttag cttaatgacc agacaccgtc ctgagtgtat tacttttcaa cagatcaagg 720
ataattgcgc taatgagctt gatctgctgg cgcagaagta ttccatagag cagctgacca 780
cttactggct gcagccaggg gatgattttg aggaggctat tagggtatat gcaaaggtgg 840
cacttaggcc agattgcaag tacaagatca gcaaacttgt aaatatcagg aattgttgct 900
acatttctgg gaacggggcc gaggtggaga tagatacgga ggatagggtg gcctttagat 960
gtagcatgat aaatatgtgg ccgggggtgc ttggcatgga cggggtggtt attatgaatg 1020

taaggtttac tggccccaat tttagcggta cggttttcct ggccaatacc aaccttatcc 1080

tacacggtgt aagcttctat gggtttaaca atacctgtgt ggaagcctgg accgatgtaa 1140

gggttcgggg ctgtgccttt tactgctgct ggaagggggt ggtgtgtcgc cccaaaagca 1200

gggcttcaat taagaaatgc ctctttgaaa ggtgtacctt gggtatcctg tctgagggta 1260

actccagggt gcgccacaat gtggcctccg actgtggttg cttcatgcta gtgaaaagcg 1320

tggctgtgat taagcataac atggtatgtg gcaactgcga ggacagggcc tctcagatgc 1380

tgacctgctc ggacggcaac tgtcacctgc tgaagaccat tcacgtagcc agccactctc 1440

gcaaggcctg gccagtgttt gagcataaca tactgacccg ctgttccttg catttgggta 1500

acaggagggg ggtgttccta ccttaccaat gcaatttgag tcacactaag atattgcttg 1560

agcccgagag catgtccaag gtgaacctga acggggtgtt tgacatgacc atgaagatct 1620

ggaaggtgct gaggtacgat gagacccgca ccaggtgcag accctgcgag tgtggcggta 1680

aacatattag gaaccagcct gtgatgctgg atgtgaccga ggagctgagg cccgatcact 1740

tggtgctggc ctgcacccgc gctgagtttg gctctagcga tgaagataca gattga 1796

<210> 4
<211> 29
<212> DNA
<213> Human adenovirus type 5

<400> 4
aaccagcgct accatgagca ccaactcgt 29

<210> 5
<211> 29
<212> DNA
<213> Human adenovirus type 5

<400> 5
aatggtggca accgcattgg gaggggagg 29

<210> 6
<211> 30
<212> DNA
<213> Human adenovirus type 5

<400> 6
gcacaattgg attctttgac ccgggaactt 30

<210> 7
<211> 10
<212> PRT
<213> Human adenovirus type 5

<400> 7

Ala Gln Leu Asp Ser Leu Thr Arg Glu Leu
1               5                   10

<210> 8
<211> 24

<212>  DNA
<213>  Modified Vaccinia Virus Ankara

<400>  8
cgttttgcat catacctcca tctt                                              24


<210>  9
<211>  18
<212>  DNA
<213>  Modified Vaccinia Virus Ankara

<400>  9
gcgggtgctg gagtgctt                                                     18


<210>  10
<211>  30
<212>  DNA
<213>  Modified Vaccinia Virus Ankara

<400>  10
aggcataaac gattgctgct gttcctctgt                                        30


<210>  11
<211>  22
<212>  DNA
<213>  Human adenovirus type 5

<400>  11
tgactccggt ccttctaaca ca                                                22


<210>  12
<211>  21
<212>  DNA
<213>  Human adenovirus type 5

<400>  12
tcacggcaac tggtttaatg g                                                 21


<210>  13
<211>  19
<212>  DNA
<213>  Human adenovirus type 5

<400>  13
cccggtggtc ccgctgtgc                                                    19


<210>  14
<211>  20
<212>  DNA
<213>  Human adenovirus type 5

<400>  14
gtggttgctt catgctagtg                                                   20


<210>  15
<211>  20
<212>  DNA
<213>  Human adenovirus type 5

<400>  15
tcttcagcag gtgacagttg                                                   20

<210>  16
<211>  19
<212>  DNA
<213>  Human adenovirus type 5

<400>  16
acctacgaga ccgtgtctg                                                    19


<210>  17
<211>  19
<212>  DNA
<213>  Human adenovirus type 5

<400>  17
gagccgtcaa cttgtcatc                                                    19

## Description

### Background of the Invention

This application relates to cells that permanentely sustain a heat shock cascade without undergoing apoptosis.

A heat shock casscade is triggered by heat shock proteins (HSP). HSP are a class of functionally related proteins whose expression is increased when cells are exposed to elevated temperatures or other stress. This increase in expression is transcriptionally regulated. The dramatic upregulation of the heat shock proteins is a key part of the heat shock response and is induced primarily by heat shock factor. HSPs are found in virtually all living organisms, from bacteria to humans.

Heat-shock proteins are named according to their molecular weight. For example, Hsp60, Hsp70 and Hsp90 (the most widely-studied HSPs) refer to families of heat shock proteins on the order of 60, 70 and 90 kilodaltons in size, respectively. The small 8 kilodalton protein ubiquitin, which marks proteins for degradation, also has features of a heat shock protein.

Hsp90 is the most abundant cellular chaperone, and has been shown in experimental systems to be required for ATP-dependent refolding of denatured or "unfolded"proteins.

Hsp90 may play a role in maintaining the stability and function of mutated proteins. It seems to be required for expression of mutated p53 and V-src to a much greater extent than for their wild-type counterparts.

Hsp90 is also necessary to the conformational maturation of several key proteins involved in the growth response of the cell to extracellular factors. These include the steroid receptors as well as certain transmembrane kinases (i.e., Raf serine linase, V-src and Her2). The mechanism whereby hsp90 affects theseproteins is not fully understood, but appears to be similar to its role in protein refolding. In the case of the progesterone receptor, it has been shown that binding and release of hsp90 from the receptor occurs in a cyclic fashion in concert with release of other chaperones and immunophilins and is required for high affinity binding of the steroid to the receptor. Thus, hsp90 could function as a physiologic regulator of signaling pathways, even in the absence of stress.

Adenoviruses are unenveloped (naked) double-stranded DNA viruses that infect a broad spectrum of animals. Among the best characterized members is the human adenovirus serotype 5 (Ad5). This virus is a frequent cause of common cold symptoms and infection often occurs in childhood.

Adenoviruses are amenable to genetic manipulation and replication incompetent adenoviruses, including Ad5 (GenBank accession number for sequence: AC_000008), are used as vectors in gene therapy and for therapeutic vaccination. To obtain replication incompetent viruses large regions of the genomic DNA are substituted with non-viral sequences. The missing viral functions are provided in trans by host cell lines that have been stably transfected with genes that are deleted in the vector. One of the earlier systems that were developed consist of adenoviral vectors deleted in the regulatory E1 region produced on cell lines providing the corresponding E1 proteins.

The most common cell line for this purpose is HEK 293. This cell line was generated in 1977 by transfection of fragmented adenoviral genomic DNA into primary human cells (Graham et al. 1977 in J Gen Virol 36, 59-74), well before it was recognized that adenoviruses may serve as gene therapy vectors. The resulting cell line was subsequently shown to contain nucleotides 1 to 4344 of the genomic DNA (Louis et al. 1997 in Virology 233, 423-429) which includes the E1 region; this characterization demonstrated that the E1 region can be used to immortalize and transform primary cells.

Adjacent to the E1 region follows the gene for pIX ("protein 9"; nucleotides 3609 to 4031 on the adenoviral genomic DNA). The promoter for pIX is embedded within the E1B component of the E1 region. A mechanism called promoter occlusion allows expression of pIX at a delayed-early time in the viral infection cycle at the onset of viral DNA replication with increasing copy number of viral DNA (Fessler and Young 1998 in J Virol 72, 4049-4056). Although the gene is present within the 4344 nucleotides integrated into 293 cells, pIX expression cannot be detected even with sensitive radioactive labeling methods (Spector et al. 1980 in J Virol 36, 860-871).

As described above, replication deficient adenoviral vectors have been generated by deletion of the E1 region from the viral genome. E1 products are essential for viral replication and the function of the E1 region therefore had to be provided in trans via stable E1 transgenes in the adenovirus packaging cell (such as the 293 cell line). Because of the proximity to the E1 region the pIX gene caused frequent recombination between deleted adenoviral vectors and E1 in the host genome. This recombination event generated replication competent adenovirus (RCA), a serious contamination in vector preparations. To suppress this recombination event pIX was deleted from the adenovirus genome. In the course of these experiments it was recognized that pIX stabilizes the adenoviral capsid against thermal and steric stress by increasing the interaction of the main building blocks, the hexons (Colby and Shenk 1981 in J Virol 39, 977-980; Ghosh-Choudhury et al. 1987 in Embo J 6, 1733-1739). Morphogenesis in the absence of pIX yields temperature-sensitive viruses that cannot deliver genomic DNA molecules larger than 105% of the wild type 35938 base pairs. To still allow a normal packaging capacity and thermal stability the pIX protein was stably introduced into cell lines intended as packaging cells for adenovirus vectors (Krougliak and Graham, Hum. Gene Ther. 6, 1575-1586 (1995); WO 99/57296 and Imler et al., Gene Therapy 3, 75-84 (1996)).

Also with recognition that pIX decorates the surface of virions pIX-fusion proteins have been generated with the purpose to expand the host range of adenovirus vectors or to follow morphogenesis and the intracellular migration of virus particles (reviewed by (Parks 2005 in Mol Ther 11, 19-25)).

Although clearly a structural protein, pIX also is implicated as regulatory protein for adenovirus replication. PIX is assumed to function as transactivator of transcription to augment E1A expression, a function possibly even excerted as a virokine by incoming PIX protein from the capsid at the infection step (reviewed by (Parks 2005 in Mol Ther 11, 19-25)). PIX, together with early protein E4 Orf3, also is described to interact with sub-nuclear inclusions called PML bodies (Puvion-Dutilleul et al., Exp. Cell. Res. 218, 9-16 (1995); Leppard and Everett, J. Gen. Virol. 80, 997-1008 (1999)). These are dynamic aggregates of 250 nm to 500 nm in size and proposed to be involved in regulation of cell differentiation (Wang et al., Science 279, 1547-1551 (1998)), control of apoptosis (Quignon et al., Nature Gen. 20, 259-265 (1998)), and response to viral infection (Möller and Schmitz, Arch Immunol Ther Exp (Warsz) 51, 295-300 (2003)). Deletion of pIX has no obvious effects on adenovirus replication at normal temperatures if the viral genomic DNA is below a certain size limit (Sargent et al. 2004 in J Virol 78, 5032-5037). However, morphogenesis of adenoviruses in absence of pIX yields particles that have difficulties to accomodate genomic DNA at or beyond wild type genome size and that are labile at elevated temperatures (Colby and Shenk 1981 in J Virol 39, 977-980). In the adenovirus infectious cycle pIX is expressed early independent of the late genes that express the main structural proteins. Thus it was speculated that pIX may have additional functions beyond capsid stabilization and was subsequently suggested to be involved in the interferon mediated anti-viral defenses of the cell (Rosa-Calatrava et al. 2003 in EMBO Rep 4, 969-975).

It must be underlined that pIX is neither a heat shock protein nor in any other way a stress related protein.

A permissive cell line means that it expresses a virus, that is able to circumvent the host defenses and is able to replicate. Usually this occurs when the virus has modulated one or several of the host cellular intrinsic defenses, and the host immune system. The permissive state of a host is the primary factor in determining whether a virus will cause pathological symptoms in a host.

That is one reason, why, for example, attenuated (weakened) viruses are promising vaccine candidates: upon inoculation they mimick a natural infection but allow more time for establishment of the desired protective immune response by the vaccinee. With increasing numbers of immunocompromised patients (for example, due to HIV infection) highly attenuated strains are desirable. Whereas attenuated strains still proceed towards (usually benign) infection highly attenuated strains are blocked at a cellular level even in absence of a functional immune system. A frequently used method to establish and maintain attenuation is to passage viruses on different host tissue. For example, measles and mumps viruses intended for human vaccination are passaged in primary cells, either in embryonated chicken eggs or cultures derived thereof. A new generation of vaccines is based on highly attenuated pox viruses that also depend on primary chicken cells for production. A cell line that can substitute for primary chicken cells and at the same time that even less efficiently protects itself against viral infection due to secondary manipulations such as introduction of the PIX transgene therefore provides a highly desirable substrate.

The virus investigated here is modified vaccinia Ankara (MVA). The results can be extended to other wildtype, attenuated, pseudotyped, recombinant or chimeric viruses, for example strains of the rhabdoviridae (especially rabies virus and vesicular stomatitis virus), herpesviridae (including veterinary Mareks disease viruses), poxviridae in addition to MVA such as fowlpox virus and canarypox virus, togaviridae (such as alphaviruses including Sindbis, Semliki or Ross River viruses; Venezuelan, Eastern or Western encephalitis viruses), flaviviruses (such as West Nile or Kunjin viruses) and paramyxoviridae (such as Newcastle disease virus). MVA was used in the examples because this highly attenuated virus is among the most promising viral vectors currently investigated for vaccine purposes. MVA was derived from vaccinia virus (that has a very broad host range) by 574 serial passages in primary chicken cells. This procedure has led to accumulation of mutations and deletions in the viral genome (Meyer et al. 1991 in J Gen Virol 72 ( Pt 5), 1031-1038) that can be complemented by chicken cells but not by human cells. Although MVA cannot multiply in most mammalian cells virus entry and gene expression is not impaired (Sutter and Moss 1992 in Proc Natl Acad Sci U S A 89, 10847-10851). Stimulation of humoral and cellular immunity is strong (Sutter et al. 1994 in Vaccine 12, 1032-1040), possibly superior compared to replication competent vaccinia virus because immune evasion factors that may interfere with antigen presentation in the vaccinee were interrupted by attenuation (Drillien et al. 2004 in J Gen Virol 85, 2167-2175; Liu et al. 2008 in BMC Immunol 9, 15). Safety of MVA has been demonstrated near the end of the smallpox eradication campaign (Mayr 2003 in Comp Immunol Microbiol Infect Dis 26, 423-430) and for patients with a compromised immune system in more recent studies (for example, (Cebere et al. 2006 in Vaccine 24, 417-425; Dorrell et al. 2007 in Vaccine 25, 3277-3283)).

The innate immunity investigated here is the pathway induced by triggering toll-like receptor 3 (TLR-3). The innate immunity is an evolutionary very old program defending the organism against various endoparasites at the level of the individual cell. One effect of the innate immune system is rapid induction of cell death to interfere with production of progeny pathogens. An important component of the innate immune system is recognition of molecular patterns associated with pathogens (PAP). Such patterns include lipopolysaccharides, lipoteichoic acid, unmethylated CpG DNA of bacteria, and dsRNA (Weber et al. 2006 in J Virol 80, 5059-5064), including its synthetic version poly-IC. PAPs are sensed by toll-like receptors (TLR), a family of proteins where each member has affinity for a specific pattern and communicates recognitation of this pattern into the interferon α/β cascade thus promoting cells into an anti-viral state (reviewed in (Baccala et al. 2007 in Nat Med 13, 543-551)). In this family of receptors, TLR-3 is an endosomal protein triggered if a cell incorporates dsRNA from the extracellular space (Alexopoulou et al. 2001 in Nature 413, 732-738). Such extracellular dsRNA could have been released by a neighboring cell lysed after viral infection so induction of the antiviral state is appropriate. Activation of TLR-3 causes expression of additional sensors for dsRNA via the inteferon pathways. The additional sensors are RIG-I, MDA-5 and PKR that occupy different locations within the cell that may serve as virus replication niduses. If PKR binds dsRNA it deactivates eIF-2α thus causing a block in ribosomal translation. Many viruses interfere with some of these defenses. For example, MVA blocks PKR activity with the E3L protein (Hornemann et al. 2003 in J Virol 77, 8394-8407).

The heat shock pathways investigated here lead to the central Hsp70/Hsp90 complexes. Heat shock activates and constitutes chaperones that mediate correct folding and targeting of proteins especially (but not only) in the presence of detrimental environmental stimuli such as nutrient scarcity, elevated temperature or toxin concentration. As replication of acute viruses also is a significant burden on the cell metabolism some viruses allow induction of heat shock to improve some steps of the infectious cycle such as morphogenesis and virus egress (Aparicio et al. 2005 in Plant Physiol 138, 529-536; He 2006 in Cell Death Differ 13, 393-403). Because viruses may induce heat shock (either directly from within the infectious cycle or indirectly as cytokine release in the infected organism causes fever) the innate immune system also communicates with this pathway. For example, P58^IPK is a negative regulator of PKR (Melville et al. 1999 in J Biol Chem 274, 3797-3803). This regulator is modulated by heat shock proteins Hsp40 and Hsp70: Hsp40 inhibits P58^IPK so that the cell reacts more readily to dsRNA whereas Hsp70 strengthens the protective activity of P58^IPK. Viruses interfere with these regulatory pathways as well; for example, influenza virus sequesters Hsp40 (Melville, et al. 1999 in J Biol Chem 274, 3797-3803) to increase the pool of activated P58^IPK and vaccinia virus benefits from the interaction with Hsp90 (Hung et al. 2002 in J Virol 76, 1379-1390). Hsp90 is central to the replication of many viruses (Connor et al. 2007 in Virology 362, 109-119), to proliferation of tumors but also in normal cellular processes (Nollen and Morimoto 2002 in J Cell Sci 115, 2809-2816). As a buffer for phenotypic changes Hsp90 has been implicated in stabilizing the genotype by alleviating selection pressures within certain limits (Queitsch et al. 2002 in Nature 417, 618-624).

A problem of the so far known cell-lines is, that the productivity steeply declines after some time due to the stress, under which the cells are put. This stress leads ultimately to apoptosis. Dependence on primary cells therefore further complicates production especially in large vaccination programs. With primary cells it is also not possible to create packaging lines for replication-deficient vectors that are adapted to proliferation in an avian host.

The applicants of US20010044146 ("Method for enhanced protein stabilization and for production of cell lines useful for production of such stabilized proteins") solved this technical problem by transiently stimulating the production of permissive eucaryotic cell lines through transiently stimulating the expression of one or more cellular stress proteins. More specifically, stress factors employed to stimulate the virus production of permissive eucaryotic cell lines presented in US20010044146 included thermal stress (such as increased or reduced incubation temperature), chemical stress (such as increased or reduced pH), oxidation level stress (such as increased or reduced levels of O₂), nutrient modification (such as reduction or enhancement of essential culture media components), and chemically agents (such as exposure to cytotoxic substances).

Although virus production could be increased by such methods the transient stimulation of stress proteins in producer cell lines has several major drawbacks:
The stimulation is only transiently and for a limited period of time of several days only.
The stimulation of cellular stress by one or more of the above methods leads to the induction of cellular apoptosis which makes it impossible to propagate the cells for a few days only.

Thus, the major drawback of transiently and unspecifically inducing stress in a cell is that the fraction of cells surviving the stress period and therefore remaining as potential producer cells inside the production system is dramatically reduced by the application of transient stress. Furthermore, those cells which would be the highest producers of foreign proteins (i.e. which have activated most stress cascades) are also those which have the lowest chance to survive in the cell pool. Thus, although the yield of foreign protein (e.g. MVA produced on average by a cell) is in general increased through the application of transient stress, the system works only at low yields and for a very limited period of time. In a production process such transient and less defined procedures come along with several problems:
In general there are high batch to batch variations. The cell batches are poorly defined through transient stimulation of the cells. The culture conditions are very variable through variable cell dividing cycles. There is a high yield of dead cells throughout the whole production process which makes it necessary to employ special purification methods to separate the proteins / virus of interest from cellular debris and cellular proteins. In case of chemical stress stimulants the final product may be contaminated with cytotoxic compounds. By employing physical stress inducers such as higher or lower temperature, a misfolding of the final protein product, and thus a reduced biological activity, may be the consequence.

Despite the fact that transiently inducing stress to a eukaryotic cell leads to a transient increase in virus production, the above disadvantages and especially the fact that such cells do not survive for a prolonged time period, make it impossible to employ such a method for generating high yield producer cells under GMP conditions.

Hence, with respect to high yields under defined culture conditions the problem can only be solved if optimal stress conditions exist under which cell viability is not reduced and stress induction is performed under stable and highly defined conditions.

The problem solved by the application at hand is therefore how to obtain cells that can be permanantely stressed without undergoing apoptosis while producing a protein or virus.

### Summary of the Invention

In the present invention, a method is described that allows the specific induction of stress in cells through the activation of the HSP90 complex. Furthermore, a method is provided that allows the permanent, instead of only a transient, activation of stress proteins through the stable transfection of cells with an expression vector carrying the adenoviral pIX gene

In short in can be stated that cell lines that have been stably transfected with adenoviral gene pIX (or a chimaeric fusion analogue thereof), are capable of permanently sustaining a heat shock cascade without going into apoptosis.

A further objective of the invention at hand is a method of testing for inhibitors of the heat shock cascade. As the cells of the invention are more susceptible against inhibition of the heat shock cascade and can therefore be used as a cellular high throughput assay.

### Short Description of the Figures

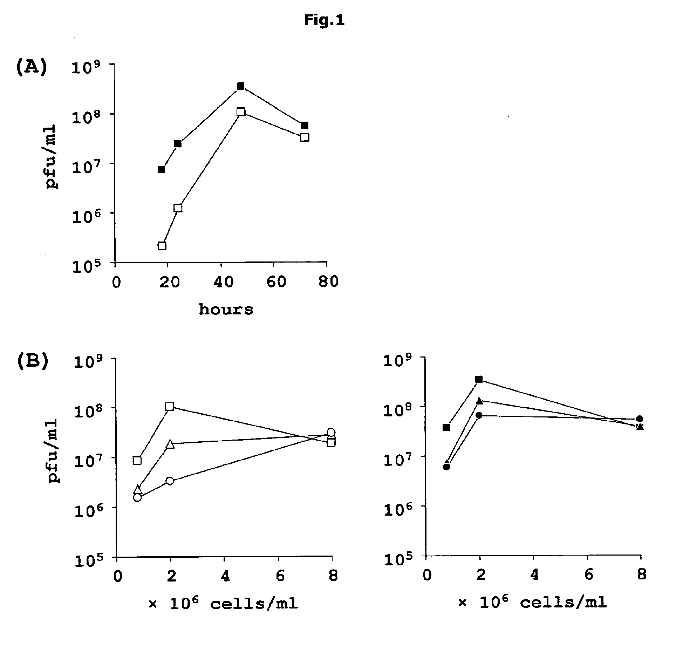

Fig. 1: . MVA production in CR cells (open symbols) and CR.pIX cells (bold symbols) adapted to suspension in media free of animal derived components. In (A), cells at 2 x 10^6/ml were infected with an MOI of 0.01 and yields were determined at the indicated time points. In (B), cells were infected with increasing MOIs at the indicated cell densities. Yields were determined 48 h post infection. Squares (■) for an MOI of 0.01, triangles (▲) for an MOI of 0.05 and circles (●) for an MOI of 0.1. Yields for MVA are increased upon expression of pIX.

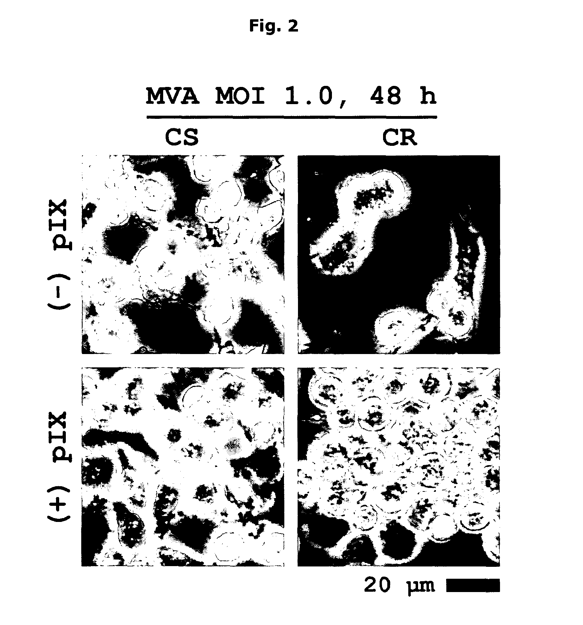

Fig. 2: PIX delays cytopathic effect of MVA in CR and CS cells. CS and CS.pIX, and CR and CR.pIX cells were massively infected with MVA at an MOI of 1.0. Onset of cytopathic effect appears delayed (or extent less severe) in the presence of pIX. This effect is best visible approximately 48 hours post infection. All lineages (including pIX-positive cultures) are susceptible to virus to similar degrees as complete lysis is evident at 72 h post infection.

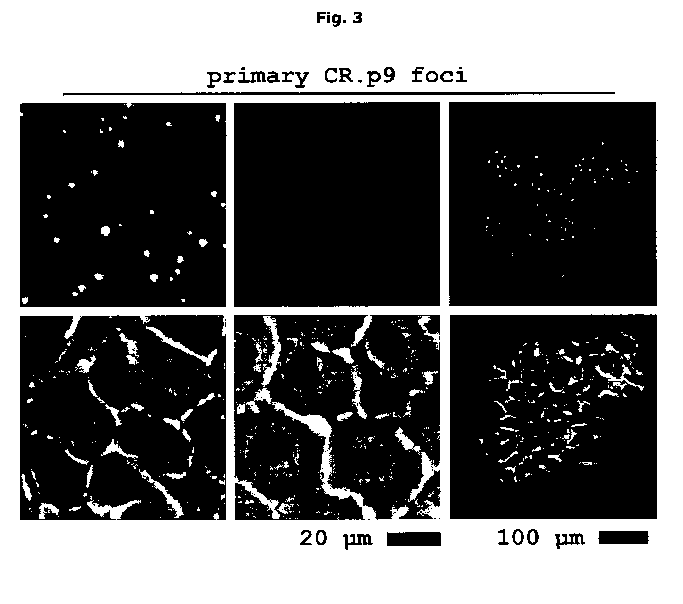

Fig. 3: Subcellular distribution of PIX-GFP in duck cells. GFP-tagged PIX appears in few cytoplasmic bright spots and diffuse cytoplasmatic staining largely excluding the nucleus in the majority of the clones. Fewer clones exhibit stronger, diffuse cytoplasmatic staining and more intense accumulation next to the nucleus.

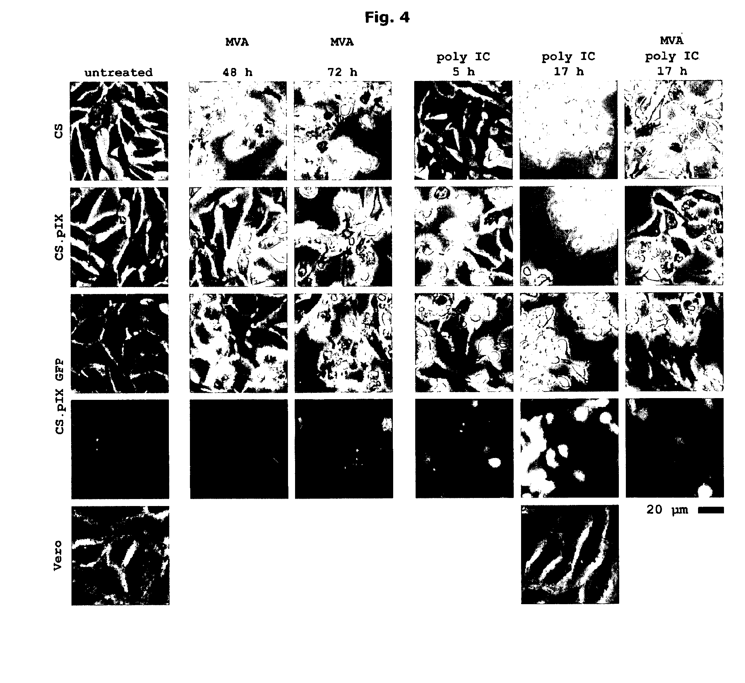

Fig. 4: Pathogen associated molecular patterns have greater effect in presence of pIX. First column is untreated reference, the next two columns deonstrate delay of MVA CPE at various MOIs also in presence of pIX-GFP. Upon addition of poly-IC (as pathogen associated pattern) cells that express pIX or pIX-GFP show greater toxic effects than parental cells within 5 h of treatment. If cells first are infected with MVA followed by addition of poly-IC induction of cell death is delayed at least 17 hours also in presence of pIX or pIX-GFP.

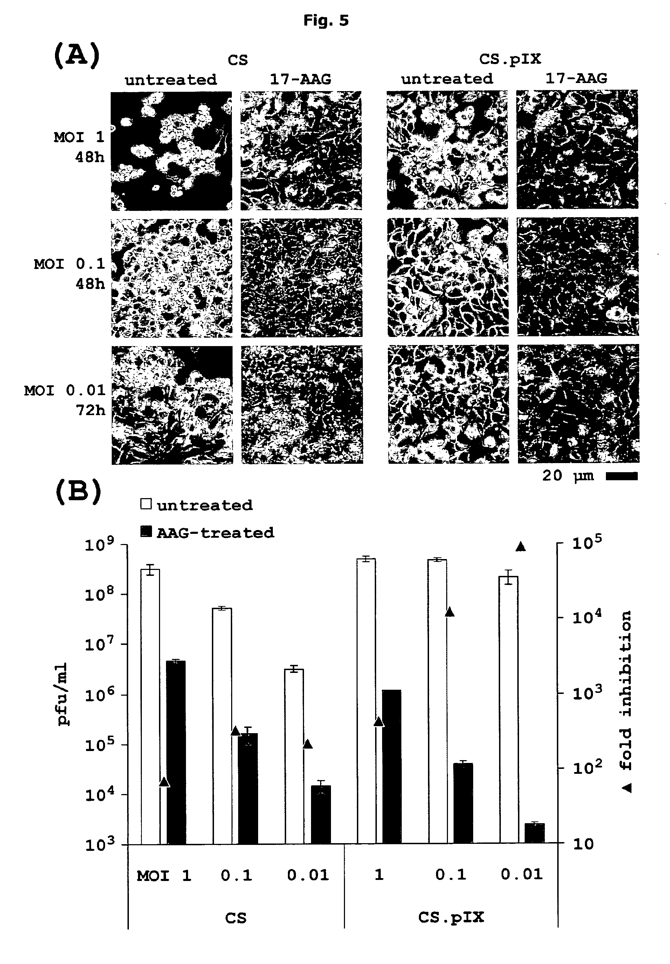

Fig. 5: Effect of 17-AAG (analog of geldanamycin with greater stability) on MVA yields in cells that express pIX: cells were infected with indicated MOI and treated with 0.5 µM 17-AAG. As shown in (A) 48 h post infection, 17-AAG at 0.5 µM is not toxic for either cell line and interferes with MVA replication. Yields of the indicated cultures are depicted in the bar chart in (B); all determinations were performed in triplicates. The triangles reside on the secondary axis and mark reduction in titer upon treatment with 17-AAG.

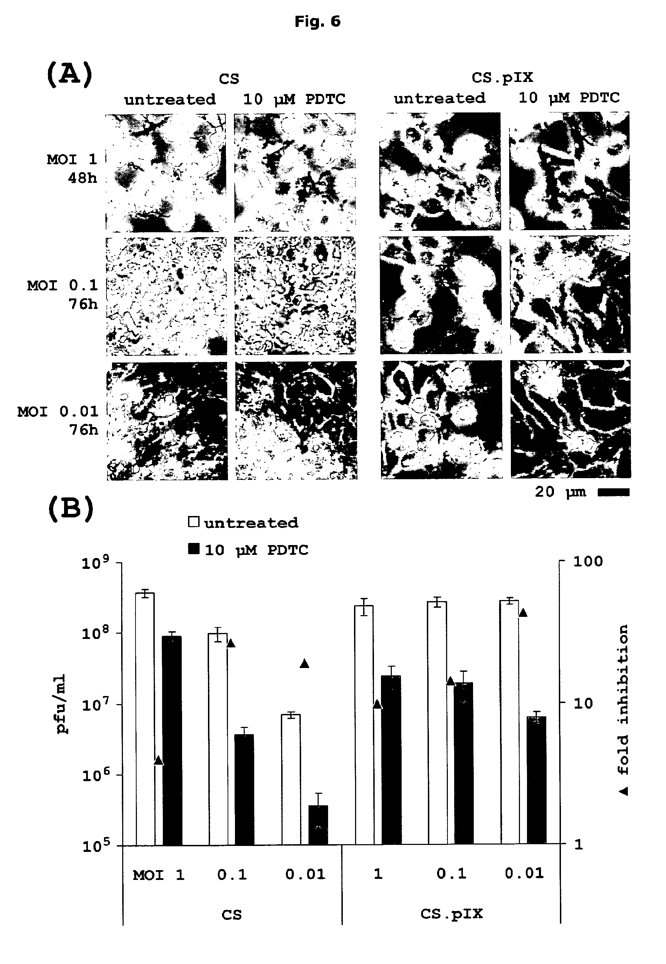

Fig. 6: Effect of NF-κB inhibitor PDTC is not influenced by pIX.

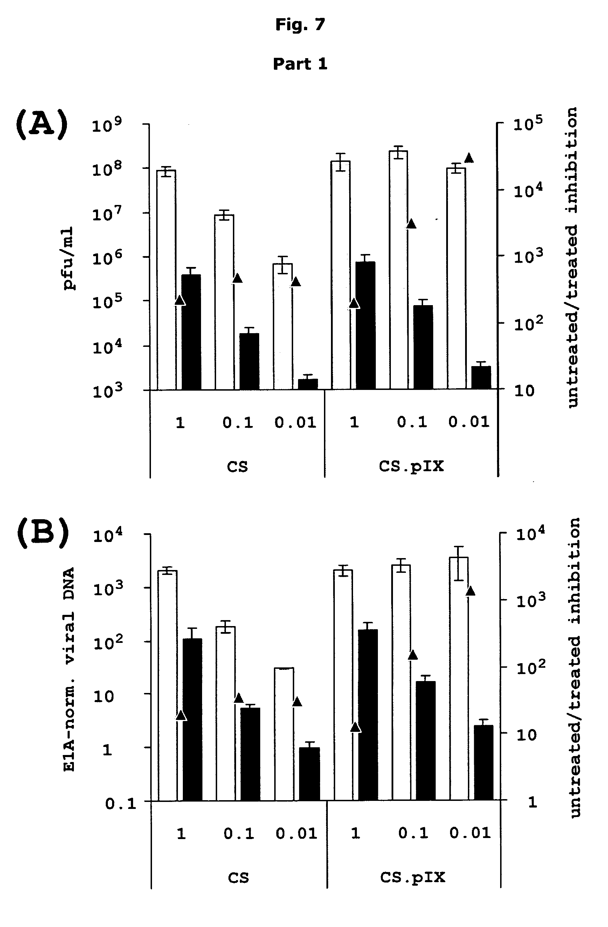

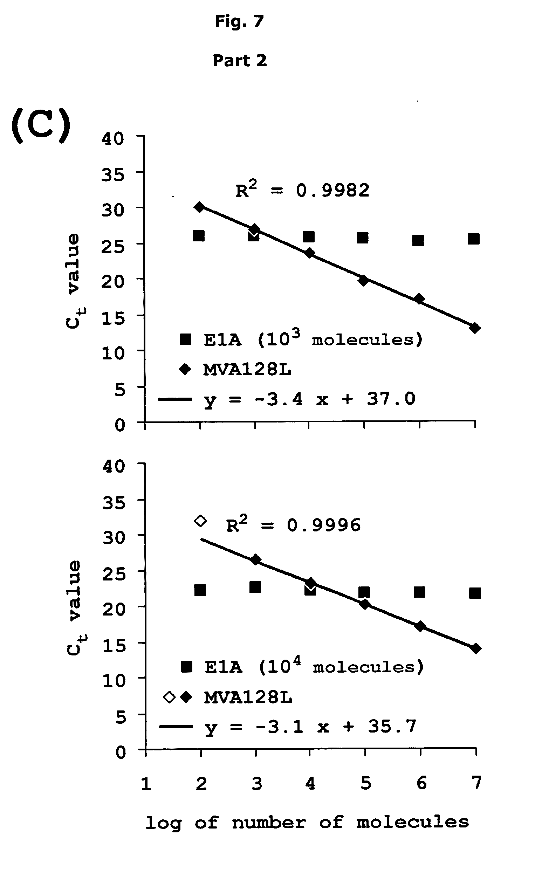

Fig. 7: pIX appears to stabilize a viral polymerase. (A) Geldanamycin treatment reduces viral titers in pfu/ml to a greater amount in pIX-positive cells confirming the data in shown in Fig. 5. (B) Geldanamycin treatment reduces MVA genomic DNA 10-fold already upon infection with an MOI of 1. (C) Standard curves to obtain PCR efficiencies for calculation of genome copy numbers.

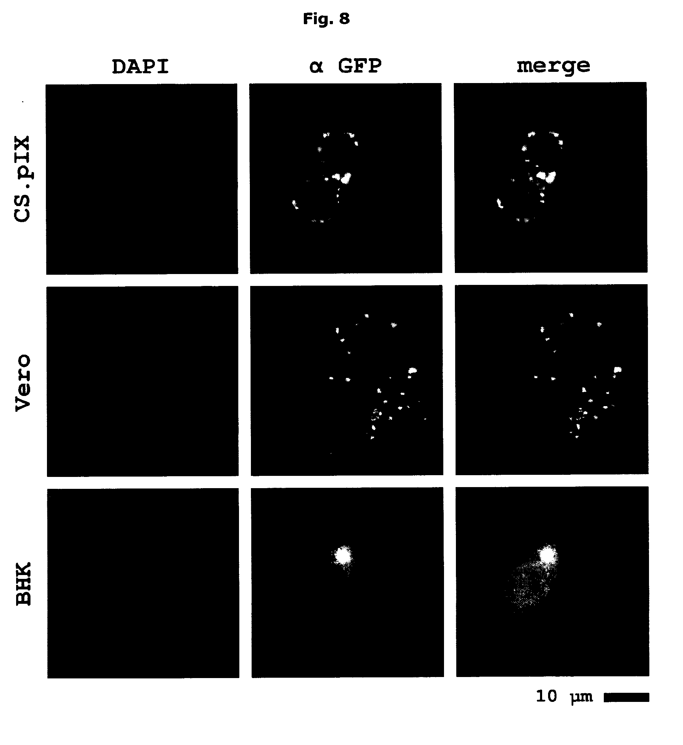

Fig. 8: pIX-GFP induces patterns consistent with stress granules in different cell types of different species.

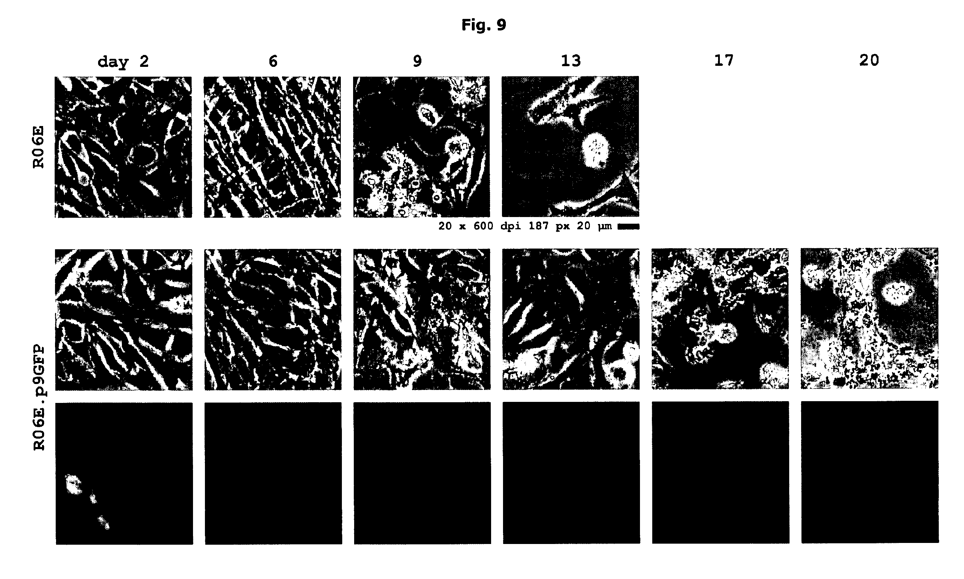

Fig. 9: pIX-GFP mediates delay of MVA CPE also in a mammalian cell line permissive for MVA. The bat cell line R06E stably expressing the PIX-GFP protein also exhibits significantly delayed CPE compared to parental reference.

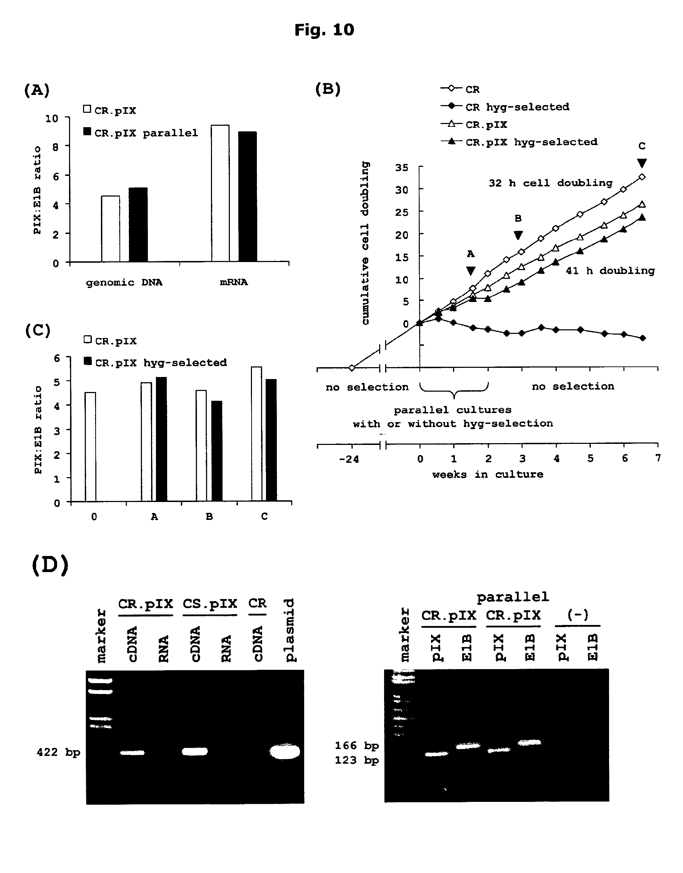

Fig. 10: pIX is stably maintained even without selection. Stable maintenance of PIX transgene in duck retina cells. (A) A clone of CR.pIX suspension cells was cultivated in parallel aliquots for > 3 months without selection. After this time, TaqMan PCR was used to count the copy numbers of E1B and pIX transgene in cells from these two lineages. As E1B transgene is maintained independent of pIX the ratio of the two genes is an indication of maintenance of pPIX transgene. The ratio remained constant. Furthermore, the ratio of pIX mRNA to E1B mRNA also did not change and pIX mRNA was in excess of E1B mRNA clearly indicating expression of pIX. (B) and (C) CR.pIX and parental (pIX-negative) CR suspension cells were cultivated with or without hygromycin selective pressure for 2 weeks. At various time points, genomic DNA was isolated (indicated by "A", "B", and "C") and subjected to real-time PCR quantification (C). Whereas parental cells where killed by hygromycin the CR.pIX cells survived. As hygromycin resistance is coupled to pIX expression via an internal ribosome entry site on a bicistronic mRNA hygromycin resistance indicates pIX expression. The ratio of transgenes remained constant, again indicating stable maintenance of pIX also in presence of selective pressure. Doubling time for parental cells is approximately 32 h for parental CR but 41 hours for the derived CR.pIX cells.

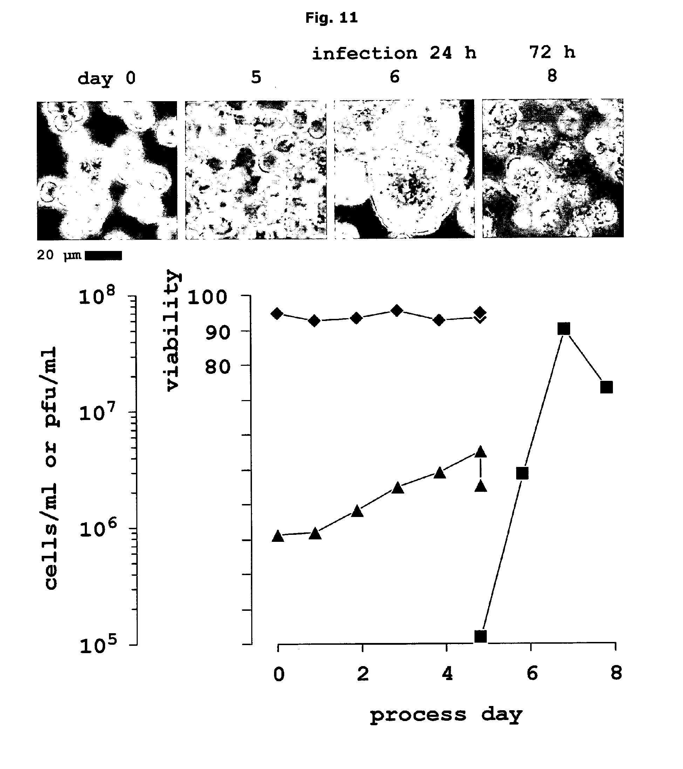

Fig. 11: pIX-positive cells are suitable for industrial processes. Shown are micrographs of CR.pIX suspension cells and proliferation profile in a DASGIP bioreactor. 5 days after inoculation of the bioreactor, cells were infected with MVA to an MOI of 0.05 in a fed-batch procedure with one volume of fresh medium containing virus.

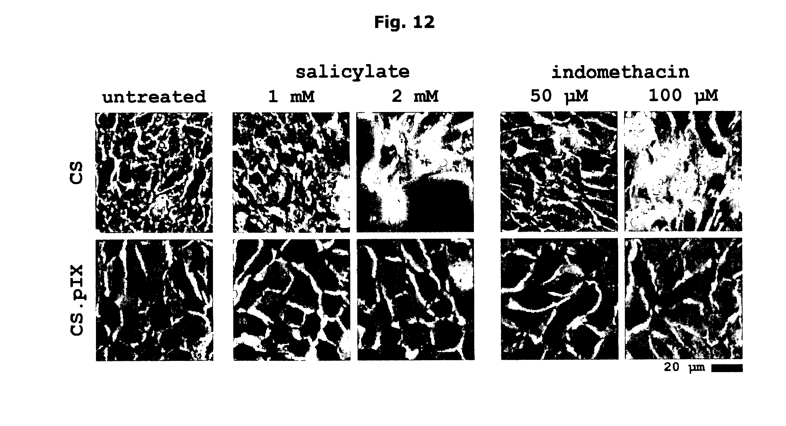

Fig. 12: pIX protects cells against environmental stress. Treatment with salicylate and indomethacine is toxic to CS cells. Toxicity is greater for pIX-negative cells.

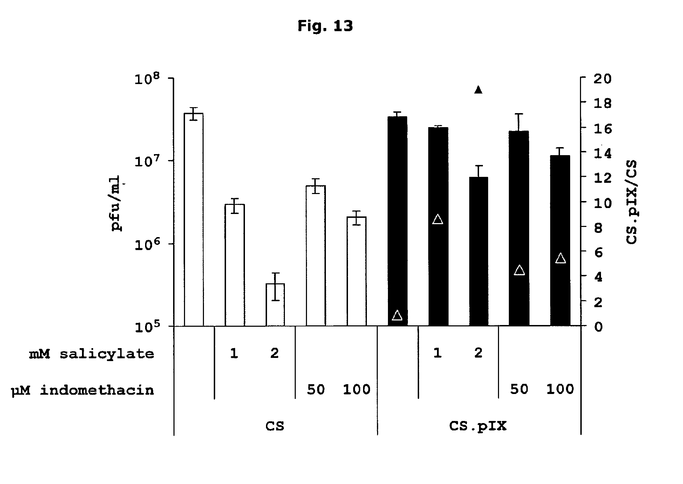

Fig. 13: pIX protects products from cells against environmental stress. Cells treated with salicylate and indomethacine were infected with MVA at an MOI of 0.1 and assayed for pfu/ml after 48 h post infection. Note that MVA titers are at least five-fold higher in presence of pIX even at sub-toxic levels of drugs (see Fig. 12; no toxic effects visible in cells treated with 1 mM salicylate or 50 µM indomethacin).

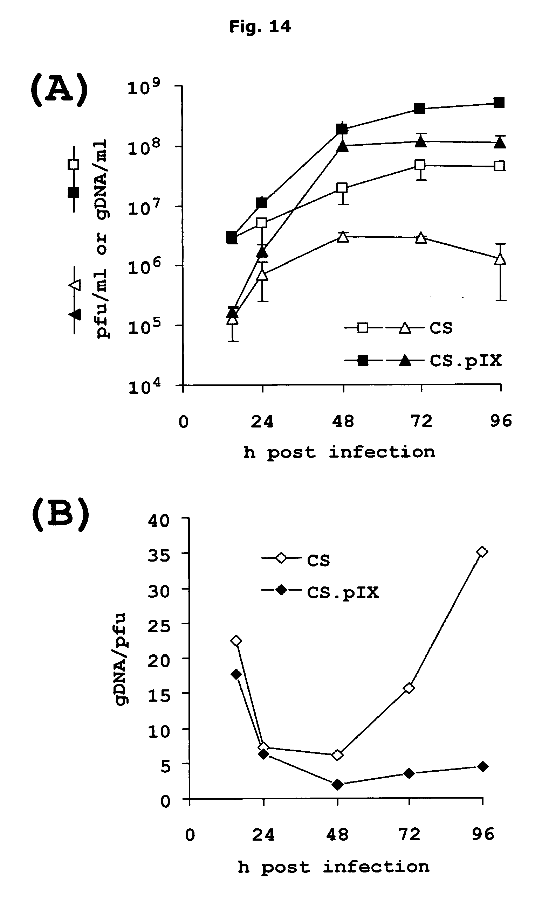

Fig. 14: Amount of viral genomic DNA required to package infectious particles is lower in presence of pIX. Panel (A) shows apparently congruent curves for pfu/ml and normalized viral genomic DNA (gDNA)/ml. Panel (B) shows ratio of pfu/ml and gDNA/ml suggesting a greater number of unpackaged DNA in absence of pIX.

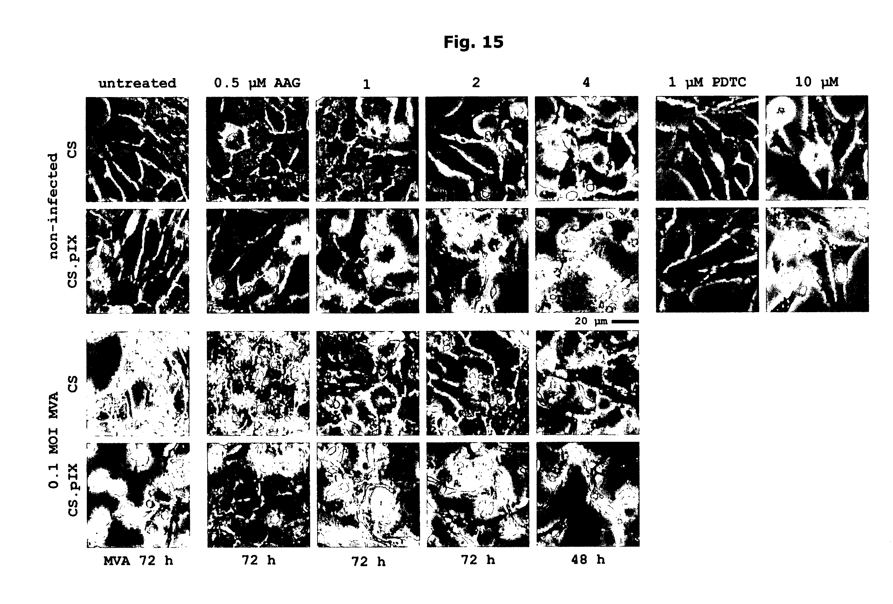

Fig. 15: Robust cell based screening procedure for inhibitors of heat shock.

### Detailled Description of the Invention

The method of embodiment (1) of the invention utilizes an expression cell having integrated into its genome a gene coding for a heterologous regulator protein, preferredly pIX or a functional variant thereof. Said regulator protein possesses the following properties:
1. It is able to permanently sustain a heat shock cascade without going into apoptosis
2.It modulates transcription and, especially if linked to appropriate modulators or regulators, also influences cell growth.
3. It enhances productivity of the cell line with regard to the production of a virus not containing said regulator protein (i.e. the regulator protein is not a substitute for a protein deleted from the virus) and/or with regard to the production of a protein differing from said regulator protein or the functional variant thereof.

According to the invention in a preferred embodiment the heterologous regulator protein is the adenovirus serotype 5 protein pIX (e.g. that having the aa sequence shown in SEQ ID NO: 1), mutants (including addition, substitution, and/or deletion mutants) thereof, variants thereof (e.g. variants obtained from an adenovirus of a different serotype), and the like.

"Functional variant of the heterologous regulator protein" according to the invention include homologues from adenovirus other than serotyp 5, all types of mutation (addition, substitution and/or deletion) of particular amino acid residue(s) of the respective wild-type regulator protein, modification by fusion that further active protein or peptide sequences and the like. Particularly preferred are fusion proteins, namly fusion proteins comprising at least one first domain comprising a regulator protein as defined hereinbefore and at least one second domain comprising a protein or peptide acting as a transcription modulator. In a preferred embodiment of the invention said transcription modulator is a transcription factor including the retinoic acid receptor alpha, which may be present in complete or incomplete (i.e. truncated) form. The modulator may also be a transit peptide, which includes NLF sequences. According to the invention the first and second domain(s) are either directly or via a peptide linker covalently attached to each other. Suitable peptide linkers include flexible and hydrophilic structures such as poly gly-ser.

It is preffered, that the heat shock cascade is triggered by HSP90.

The terms "cell" and "cell line" as used in the following description refer to expression cells/expression cell lines and host cells/host cell lines.

According to the invention it is preferred that the heterologous regulator protein or the functional variant thereof is expressed in the cell in an amount of at least 1 pg/µg cellular protein, preferably at an amount of at least 10 pg/µg cellular protein so that its expression can be determined by Western-blotting.

In the cell of the invention the heterologous regulator protein or the functional variant thereof is preferably under control of a stabile homologous or heterologous promoter.

According to the invention the cell is a vertebrate cell including mammalian cells, avian cells and the like. Suitable mammalian cells are human cells, and rodent cells including mouse, rat, hamster, etc. Particularly preferred mammalian cells according to the invention are cells derived from human brain, particularly from human foetal brain, a mouse NSO or Sp2/0 cell, a BHk or CHO cell. Suitable avian cells are duck, chicken quail and goose cells. Particularly preferred avian cells according to the invention are cells derived from a duck retina-derived or somite-derived cell.

On the other hand, the cell of the invention may be derived from a primary cell or from a previously immortalized cell. Moreover, the cell may carry further immortalizing (viral) genes including an E1 protein of an adenovirus, such as the E1 protein of mastadenovirus group C type 5, and the like. Particularly preferred according to the present invention is that the cell further expresses the adenovirus E1A and/or E1B coding sequences shown in SEQ ID NO: 2 and 3.

The cell utilized in the method of embodiment (1) of the invention may further carry functional sequences, e.g. sequences required for its application as expression cell such as selection marker sequences, splice donor/acceptor sites and/or recombinase recognition sequences allowing integration of a target nucleic acid sequence to be expressed in the cell, etc.

The "infecting", "transfecting" and "transforming" effected within the methods of the invention may be performed according to standard procedures known to the skilled person. Said methods may further encompass suitable selection, isolation and expansion steps, if required.

In the methods of the invention the target virus includes from wild-type, mutated or deleted virus, cold adapted or attenuated virus, vaccine strains, viral vectors carrying heterologous gene(s), viral vectors such as lentivirus, poxvirus, e.g. Modified adeno associated virus (aav), herpesvirus, flavivirus and the like. In particular rhabdoviridae (especially rabies virus and vesicular stomatitis virus), herpesviridae (including veterinary Mareks disease viruses), poxviridae, in particular MVA, fowlpox virus and canarypox virus, togaviridae (such as alphaviruses including Sindbis, Semliki or Ross River viruses; Venezuelan, Eastern or Western encephalitis viruses), flaviviruses (such as West Nile or Kunjin viruses) and paramyxoviridae (such as Newcastle disease virus)

Further, in the methods of the invention the one or more target proteins include antibodies, recombinant proteins such as erythropoetin, alpha-1-antitrypsin, blood clotting factors VIII and IX and interferons, viral antigens such as influenza HA and NA, and M, HBV-S, herpes G protein and rabies G protein, peptid hormones and the like. Although the method allows the production of cells capable of contemporary expression of more than one target proteins, it is particularly preferred that it encodes only one target protein.

The culturing and isolation of the methods of the invention may be performed according to standard procedures readily available to the skilled person. Said method may further include standard purification steps as well as subsequent modification steps of the target virus or target protein(s).

The cell line NC5T11#34 was deposited with the DSMZ-Deutsche Sammlung von Mikroorganismen und Zellkulturen GmbH, Mascheroder Weg 1b, 38124 Braunschweig, Germany on November 4, 2005 under accession number DSM ACC2744. The cell line CR.PIX (17a11b) was deposited with the DSMZ-Deutsche Sammlung von Mikroorganismen und Zellkulturen GmbH, Mascheroder Weg 1b, 38124 Braunschweig, Germany on November 24, 2005 under accession number DSM ACC2749.

The invention will be explained in more detail by means of the following examples, which are, however, not to be construed to limit the invention.

### Examples

### Example 1 Generation of duck retina cells expressing pIX

The cell line CR, derived from primary duck retina cells and immortalized according to defined risk guidelines is described elsewhere (patent application WO05042728, plasmid 60E-transfected retina cells). The cells were cultivated in DMEM:F12 medium (Invitrogen, Carlsbad, CA 92008, USA) supplemented to 5% fetal calf serum (Biochrom AG, 12213 Berlin, Germany) at 39 °C and 7.5% CO2. For passaging, the cells were briefly treated with TrypLE Express (Invitrogen).

The adenoviral pIX gene was amplified with primers AAC CAG CGC TAC CAT GAG CAC CAA CTC GT (SEQ ID NO: 4) and AAT GGT GGC AAC CGC ATT GGG AGG GGA GG (SEQ ID NO: 5) from adenovirus serotype 5 genomic DNA. Expression is under control of human elongation factor 2 promoter (EF2; located on chr19:3,935,325-3,936,638 in the human genome assembly May 2004). Via a weak internal ribosome binding element expression of pIX was linked to the expression of the hygromycin selection marker. The EF2 promoter in the expression plasmid contains upstream elements and the first intron of the EF2 gene. The natural start codon of EF2 is located 5' to the first intron and was removed by mutagenesis. The resulting plasmid is 76F pEFPIX1.

Plasmid 76F pEFPIX1 was linearized with SspI and XmnI restriction enzymes (both from New England Biolabs, Beverly, MA 01915-5599, USA) and purified to 400 ng/µl by affinity chromatography (gel extraction kit from Qiagen, 40724 Hilden, Germany). 5 µl (2 µg) of the purified DNA was transfected into CR cells with the Effectene reagents (Qiagen): DNA was mixed with 16 µl Enhancer and 200 µl EC Buffer. After 2 min at room temperature, 18 µl Effectene were added and liposome formation allowed for 10 min at room temperature. The transfection mix was added to 1 ml culture volume on cells seeded to 80% confluency in 6-well plates on the previous day. High transfection efficiency was confirmed with a parallel transfection of pEGFP-N1 (Clontech, Palo Alto, CA 94303-4230, USA).

The pIX-transfected cultures were expanded into T75 flasks three days after transfection and selection was initiated with 25 µg/ml hygromycin B (Invitrogen). Medium was replaced once per week with hygromycin B raised to 50 µg/ml. After three weeks, a total of four large foci survived and were re-seeded into a T25 flask: cells were detached with TrypLE and collected with 100 x g for 10 min, resuspended in fresh medium and plated into a T25 flask.

Four weeks after transfection, cells from a healthy, sub-confluent culture in a T25 flask were detached with TrypLE into 5 ml of DMEM:F12, 5% FCS. 2 ml thereof were mixed with medium #63032-1000M (JRH Biosciences, KS 66215, USA) a medium free of animal-derived components intended for maintenance of suspension cultures. Hygromycin B was added to 50 µg/ml. Total RNA and genomic DNA was isolated from 2 ml of the culture. RNA was reverse-transcribed into cDNA with cloned AMV reverse transcriptase (Invitrogen) according to the instructions by the manufacturer with random hexamers as primers. Genomic DNA, cDNA and non-transcribed RNA as control was tested with in PCR against the pIX transgene with primers shown in SEQ ID NO: 4 and 5; the expected amplicon is 422 bp in size. Fig. 10 in panel (D) shows a typical result of the RT-PCR in the left panel: cDNA yields the expected amplicon of 442 bp, non-transcribed RNA and cDNA from parental CR cells does not yield any amplicon. Plasmid 76F serves as a positive control. Fig. 10 (dicussed in greater detail in example 11) demonstrates stable insertion and expression of the pIX transgene in the CR cells, now termed "CR.pIX".

Expression of the PIX protein was also confirmed by Western blotting: 6x105 cells were disrupted by boiling in (20 mM Tris pH 7.4, 300 mM NaCl, 1% Nadesoxycholat, 1% Triton® X-100, 0.1% SDS) and protein was separated by gel electrophoresis, then transfered to a nylon membrane. PIX was detected with pIX primary antibody (a generous gift from Dr. W. Seidel, Ernst-Moritz-Arndt-Universität Greifswald, Germany), then reacted with secondary antibody directed against the former antibody and labeled with alkaline phosphatase. Extract from HEK-293 cells prepared in a parallel reaction served as a negative control. A signal of expected size confirms expression of pIX in CR.pIX cells.

The cells were cultivated in medium #63032-1000M for two additional passages, then transfered to medium #14561-1000M (also from JRH Biosciences) for two additional passages. All JRH media were supplemented to 1 x with Glutamax I (Invitrogen) and 100 µg/ml hygromycin B.

With the second passage in medium #14561-1000M a small fraction (approx. 2% of a 5 ml suspension culture) was resuspended thoroughly into DMEM:F12, FCS medium and plated into a 15 cm-diameter petri dish with. After 6 days, eleven foci were removed to individual cavities of a 12-well plate and maintained in DMEM:F12, FCS medium. For clone transfer, the medium was aspirated and cloning disks (Sigma, MO, USA) soaked in TrypLE, briefly applied directly to the clones, then transfered to the cavities filled with medium. Individual clones were expanded for further experiments and determination of growth properties.

### Example 2 Generation of duck somite cells expressing pIX

The cell line CS is derived from embryonal somites, also described in patent application WO05042728. Generation of PIX-positive CS cells was performed in parallel to the above described procedure for the CR line. Contrary to CRpIX, no suspension cultures were established for CSpIX. Strict anchorage dependency is a feature of the CS cells. A cell that does not proliferate in suspension can be considered to be less tumorigenic as the potential to metastasize is severly impaired. PIX protein does not change this property in CS cells. Although pleotropic this protein therefore appears not to impact on the transformation phenotype. This observation supports our assumption that PIX can safely be applied in biopharmaceutical processes. Expression of the pIX mRNA in CS.pIX is demonstrated in Fig. 10 in panel (D) with cDNA obtained from CS.pIX in an experiment already described example 1.

### Example 3 PIX effect on MVA replication in CS cells

Contrary to CS cells, the CR cells are adapted to suspension. Suspension cultures are preferred for many industrial applications.

Production of MVA in true suspension cultures of CR cells is shown in Fig. 1. Common suspension media were found to interfere with MVA production so that for the experiments described here suspension medium was replaced with SFM4MegaVir (Hyclone) without FCS at the time of inoculation with virus. Virus was added directly to the medium at the indicated MOIs.

MVA production is influenced by cell density at the time of infection consistent with a common observation that a dividing cell in the log phase of proliferation is most conducive for virus replication. Virus yield is higher if the infection is initiated with an MOI of 0.01 compared to higher MOIs. Maximum titers are obtained 48 h to 72 h post infection. We consistently observe higher titers for MVA in cells that stably express pIX compared to yields in parental cells.

To briefly describe isolation and quantification of virus: MVA was isolated from cells by three cycles of freeze/thawing of the cell suspension. The cryogenic lysate was centrifuged with 13000 g for 5 min; cellular debris was discarded and only the clear lysate was further analysed. Discarding debris reduced yields approximately 3-fold but also reduced variability in titration. Titration was performed in Vero cells (African green monkey kidney cells; ATCC CCL-81) in a variation of the procedure described by Reed and Munch (Reed and Muench 1938 in Am J Hyg 27, 493-497), briefly: serial dilutions of virus suspension were added to Vero monolayers. After 48 hours, the cells were fixed with methanol and incubated with polyclonal vaccinia virus antibodies (Quartett Immunodiagnostika, Berlin, Germany) at 1:1000 dilution in PBS containing 1% fetal calf serum. Two wash steps were performed with PBS containing 0.05% Tween 20 and secondary antibody to the vaccinia-specific antibody is added at 1:1000. This secondary antibody is coupled to the peroxidase enzyme that catalyzes a color reaction upon incubation with AEC reagent (3-amino-9-ethyl-carbozole; 0.3 mg/ml in 0.1 M acetate buffer pH 5.0 containing 0.015% H2O2). Infected foci are identified by light microscopy and plaque forming units/ml are calculated from the maximum dilution of MVA suspension that yields a positive dye reaction. All titrations were performed in parallel replicates (giving a total of four titration values per sample).

To briefly highlight the importance for improvement of already good MVA yields: because MVA cannot perform even limited replication in humans the vaccine dosis has been estimated at a high 10^8 pfu/ml for most applications (Coulibaly et al. 2005 in Virology 341, 91-101; Malkin et al. 2006 in Trends Parasitol 22, 292-295). Production of MVA therefore is challenging, especially for global programs against complex infectious diseases such as HIV or tuberculosis that may require hundreds of million of doses annually.

### Example 4 pIX effect on MVA cytopathic effect in adherent cells

We noted in the majority of the experiments a delay in cytopathic effect if pIX protein is expressed in the host cell. The differences in cytopathic effect usually are most prominent 48 h post infection. Fig. 2 shows an example. CS and CR cells stably transfected for PIX expression and parental CS and CR cells as reference were seeded into a 6-well plate at 2 x 10^6 cells/cavity and infected with MVA at an MOI of 1.0 on the following day. The images were taken 48 hours post infection.

Also at infection with low MOI (such as 0.01), 72 h to 96 h post infection all cultures were lysed completely suggesting that the differences in the extent of CPE at 48 h is not due to a mixture of MVA-refractory and MVA-susceptible cells in the CS.pIX or CR.pIX populations.

### Example 5 Nuclear exclusion of PIX

A PIX-GFP fusion gene was generated to visualize distribution of the PIX protein in life cells and to overcome weak binding activity of the available antibody: Plasmid 76F pEFPIX1 was treated with AccI und DraI restriction enzymes and termini were blunted with Klenow polymerase (all from New England Biolabs, Beverly, MA 01915-5599, USA). Out of the complex banding pattern, the desired fragment of 447 bp containing the PIX coding sequence was isolated by agarose gel extraction (gel extraction kit from Qiagen, 40724 Hilden, Germany). The DraI restriction enzyme recognizes the sequence "ttTAAa" to cut after the last thymidine residue, wherein the central "TAA" triplett is the stop codon of the PIX open reading frame. The 447 bp fragment therefore is devoid of a stop codon. Fusion of this fragment to the gene for EGFP in the plasmid pEGFP-N1 opened with SmaI (also New England Biolabs) generates a continous fusion gene of PIX followed by EGFP. The resulting plasmid was named p9GFP, the expressed fusion protein will be named PIX-GFP in this text.

CS and CR cells were transfected with p9GFP and selected with 300 µg/mL geneticin (Invitrogen) for stable PIX-GFP expression. Two populations of different PIX-GFP expression were observed within 2 weeks: one population had usually 2 to 5 bright spots of PIX-GFP in the cytoplasm, the other population had a more uniform expression of PIX-GFP in the cytoplasm and more diffuse accumulation of GFP signal in a region proximal to the nucleus. In both cases, the cell nuclei appeared as a dark region suggesting that no or very little chimeric protein enters the nucleus. Representative examples for both types of clones in CRpIXGFP are shown in Fig. 3. Consistent with this result we found a nuclear export sequence in the PIX open reading frame by applying the search algorithm NetNES (Cour et al., Protein Eng. Des. Sel. 17, 527-536 (2004)) to the PIX primary sequence. The NES identified by this program is 313-GCACAATTGGATTCTTTGACCCGGGAACTT-342 (SEQ ID NO: 6) (translated: AQLDSLTREL; SEQ ID NO: 7). To our knowledge, it is the first time that such a signal has been described in the PIX protein. Conversely, a nuclear localisation sequence (NLS) has not been described in the literature for PIX and we cannot detect such a signal (for example, using the algorithm provided by the Columbia University at http://cubic.bioc.columbia.edu/cgi/var/nair/resonline.pl). The cytoplasmic localisation of PIX-GFP in our duck cells therefore is consistent with the primary sequence of PIX.

### Example 6 pIX activates HSP90

Although stable expression of pIX delays CPE in the infected cells (Fig. 4, see column with MVA 48 h after infection) we unexpectedly observed an increased sensitivity to dsRNA (or synthetic analog poly-IC). The synthetic dsRNA was added directly to the medium at a concentration of 50 µg/ml. Surprisingly, cell death is induced faster in the presence of pIX (Fig. 4, see column with cells 5 h after induction). Phlebbing of the cells and the nuclei in DAPI staining (data not shown) is consistent with induction of apoptosis. In the co-evolution of virus and host many viruses have developed strategies interfering with the innate immune response. MVA resists induction by dsRNA via the viral E3L protein. Thus, to confirm that increased sensitivity to poly-IC is not due to mere cytotoxicity cells were first infected with MVA and then treated with poly-IC (Fig. 4, last column). Indeed, cell death induced by poly-IC is less severe in cells infected with MVA. The last two rows of Fig. 4 demonstrate that the cytoplasmic pIX-GFP causes delays in CPE and increased sensitivity to poly-IC indistinguishable from wild type pIX. Bottom row with Vero cells is a negative control for poly-IC cytotoxicity: 100 µg/ml do not affect Vero cells as expected as these cells are defective in interferon response (Emeny and Morgan 1979 in J Gen Virol 43, 247-252). Note also delay in CPE at 48 h in untreated controls if pIX or p9GFP is expressed.

The above experiment together with the following serendipitous observation provided the indication that pIX is involved in heat shock modulation: to examine an antiviral compound (17-AAG, a cyclic analog of geldanamycin with decreased cell cytotoxicity) in presence or absence of pIX we unexpectedly observed that although pIX increases MVA titers across a wide range of MOIs (see light-shaded bars in chart of Fig. 5 with untreated cells), cells expressing pIX suffered a more drastic decrease in MVA titers upon treatment with 17-AAG. 17-AAG is an inhibitor of activated (rather than latent) Hsp90. If pIX causes activation of Hsp90 then 17-AAG is expected to interfere to a greater extend in cells that stably express pIX.

Fig. 5 demonstrates inhibition of MVA replication in presence of 17-AAG with little concomitant cytotoxic effect from the drug. If pIX activates Hsp90 to support replication of MVA the inhibition by 17-AAG should be stronger in the presence of this viral factor. Indeed, titration of progeny virus in cell lysates reveales greater interference with virus release by 17-AAG in the presence of pIX. For example, after infection with an MOI of 0.01 treatment with 17-AAG reduces titers 200 fold in CS cells and 92000-fold in CS.pIX cells.

In summary: pIX presents a paradox in that it increases titers of unrelated pox viruses in a host cell that is rendered more susceptible to induction of anti-viral mechanism. This paradox is resolved if (as we found by accident using geldanamycin) pIX induces a cellular program that is beneficial for the virus but also a trigger for antiviral mechanism. The involved cellular program is the heat shock response, more specifically activation of Hsp90 probably by actively recruiting Hsp70 towards activation of Hsp90.

As viral infection is a significant burden on the cell some viruses induce heat shock to support the infectious cycle. Because viruses may induce heat shock the innate immune system also communicates with this pathway. For example, P58^IPK is a negative regulator of PKR (Melville, et al. 1999 in J Biol Chem 274, 3797-3803). This regulator is modulated by heat shock proteins Hsp40 and Hsp70: Hsp40 inhibits P58^IPK so that the cell reacts more readily to dsRNA whereas Hsp70 strengthens the protective activity of P58^IPK. Viruses interfere with these regulatory pathways as well; for example, influenza virus sequesters Hsp40 (Melville, et al. 1999 in J Biol Chem 274, 3797-3803) to increase the pool of activated P58^IPK, and vaccinia virus benefits from the interaction with Hsp90 (Hung, et al. 2002 in J Virol 76, 1379-1390).

It appears from our experiments that pIX also augments the heat shock response to aid in virus maturation thus increasing MVA titers. Upon challenge with geldanamycin inhibition of MVA is 400-fold greater in the presence of pIX (following infection with an MOI of 0.01 titers are reduced 200 fold in CS cells and 92000 fold in CS.pIX cells). This observation suggests that pIX promotes Hsp90 into an activated state because geldanamycin at physiological concentrations has higher affinitiy for activated (rather than latent) Hsp90 (Kamal et al. 2003 in Nature 425, 407-410). Hsp90 requires cofactors for activity including Hsp40 and Hsp70 (Kosano et al. 1998 in J Biol Chem 273, 32973-32979). Hsp70 is known to interact with the coiled-coil domain of the cellular protein IKK-gamma (Ran et al. 2004 in Genes Dev 18, 1466-1481). pIX also contains such a domain (Vellinga et al. 2005 in J Virol 79, 3206-3210). Thus, we may have discovered and utilize a function in pIX beyond mere self-aggregation.

### Example 8 pIX is specific in its activity

From the literature it could be inferred that the coiled-coil domain of pIX competes with the Hsp70-interacting coiled-coil domain of IKK-γ (Ran, et al. 2004 in Genes Dev 18, 1466-1481). Interference with IKK-gamma is expected to modulate NF-κB activity, but not to activate NF-κB by itself. The effects of NF-κB activation (especially in context of a viral infection (Santoro et al. 2003 in Embo J 22, 2552-2560) often are very complex. We know from the literature that MVA (contrary to the less attenuated parental vaccinia virus) induces NF-κB activity which may eventually lead to apoptosis (Gedey et al. 2006 in J Virol 80, 8676-8685). Since we observe delayed CPE and prolonged production of virus in pIX-positive cells infected with MVA (see Fig. 2, 4 and 14) it appears that the competition with IKK-γ is beneficial.

Thus, from Hsp70 interaction it is not expected that NF-κB activity is elevated in the naive (non-infected, unstressed) cell. However, other stressors leading to heat shock activation often also activate NF-κB. Interaction of the heat shock response and NF-κB cascades is highly complex with variable effect on strength or type of NF-κB signaling (Beere 2005 in J Clin Invest 115, 2633-2639; Salminen et al. 2008 in Immunol Lett 117, 9-15). To further demonstrate that pIX does not activate heat shock via unspecific stress pathways we next questioned the cells for NF-κB activity in naive (Fig. 15) and in infected cells (Fig. 6) using pyrrolidine dithiocarbamate (PDTC; Sigma). PDTC as an inhibitor of NF-kB activity interferes with replication of some viruses (Gaudernak et al. 2002 in J Virol 76, 6004-6015).

In our experiments, PDTC treatment at concentrations below cytotoxicity (5 pM, data not shown) or just at cytotoxicity for both cell lines (10 µM, shown in Fig. 6) reduced MVA titers but without obvious dependence on the presence or absence of pIX. PDTC was added directly into the medium at the time of infection.

This finding suggests that the activity of pIX is very selective for Hsp70 and Hsp90. This finding also suggests that a persistent (detrimental) modulation of NF-κB that would occur by proteotoxic stressors is not mediated by pIX.

### Example 9 pIX improves quality of product

Specific activity is considered the quantifiable biologic effect of a product related to a given amount. The means of production has a high impact on specific activity. Biomolecules are defined by amino acid and nucleic acid sequences but also by structural and biochemical features. Missfolded proteins contribute to the amount but not to the activity. For a virus the specific activity is described as ability to productively infect cells, e.g. to form plaques on a cell monolayer, lyse cultured cells, express genes (viral or recombinant) or cause a pathologic effect related to the amount of virus particles or its nucleic acid. Mutant, defective or incomplete particles lower the specific activity. It is desired to produce biological products with very high specific activity, e.g. intact proteins and intact viruses with unchanged sequence and conformation.

Often measurement of yield is performed in a functional assay. For example, for MVA plaque forming units are determined instead of counting MVA virions by electron microscopy or other means. Thus, if yield in a functional assay increases this may also be due to improved specific activity of a product. In our example with MVA, a pIX-positive producer cell may release the same number of virus particles compared to the parental host cell but infectivity per particle is higher if the virions have been packaged in presence of pIX-augmented heat shock.

To determine whether this effect may explain increased titers in presence of pIX we quantified the amount of viral genomic DNA that is produced in infected cells of the various lineages. Amount of genomic DNA is a non-functional correlate for virus particles. To increase precision of this assay real-time PCR measurement of viral DNA and cellular DNA as reference was performed in a multiplex assay in a single reaction tube.

We challenged virus replication with 17-AAG to test heat shock involvement in particle formation. As 17-AAG preferentially targets activated Hsp90 a greater inhibition denotes greater dependence on (and benefit from) activated Hsp90. Fig. 7 shows in panel (B) that 17-AAG interferes with DNA replication to a greater extend in presence of pIX than in absence of pIX. Tenfold decrease of amount of genomic DNA at an MOI of 1 (thus, in a single generation of virus) suggests that viral replication complex benefits significantly from the presence of pIX. Panel (A) is a control and (C) further explains the procedure: adherent cells (CR and CR.pIX (not shown) or CS and CS.pIX) in DMEM/F12 containing 5% FCS were infected with MVA at the indicated MOIs. Cell lysates containing cellular debris and free and cell-associated virus were harvested at full CPE (48 h to 72 h post infection). Virus was liberated from cell association by three freeze/thaw cycles and this cryogenic lysate was separated into two aliquots. One aliquot was analyzed by titration for pfu/ml on Vero cells as described above (results shown in panel (A)). From the other aliquot DNA was isolated using a silica matrix (Qiagen DNA extraction). 10 µl (12.5% of the yield) of this total DNA was subjected to real time PCR analysis using primers that recognize the 128L gene of MVA (bp 120811-120898, IMV membrane protein) and the E1A transgene in the cells. PCR was performed in 25 µl final volume with 100 nM of each of the four primers and 80 nM of the probes against the two amplicons, 200 µM dNTP mix, forward Primer MVA, TaqMan Universal PCR Master Mix to 1 x concentration. The primer/probe combinations were CGT TTT GCA TCA TAC CTC CAT CTT (SEQ ID NO: 8), GCG GG TGC TGG AGT GCT T (SEQ ID NO: 9) and 6FAM-AGG CAT AAA CGA TTG CTG CTG TTC CTC TGT-BHQ1 (SEQ ID NO: 10) for MVA; TGA CTC CGG TCC TTC TAA CAC A (SEQ ID NO: 11), TCA CGG CAA CTG GTT TAA TGG (SEQ ID NO: 12), and YAK-CCC GGT GGT CCC GCT GTG C-BHQ1 (SEQ ID NO: 13) for E1A.

In previous experiments with cloned 128L and E1A PCR efficiencies were determined according to the algorithm of Pfaffl (Pfaffl 2001 in Nucleic Acids Res 29, e45). Panel (C) shows a typical standardization result: number of recombinant MVA 128L molecules was decreased in steps of 10-fold dilutions from 10^7 to 10^2 molecules, the number of recombinant E1A molecules was kept constant at 10^3 or 10^4, respectively. The log of the number of 128L templates is plotted against the Ct values. The slope of the regression curve yields efficiency E of the PCR reaction: E = 10^(-1/slope). A value of 2.0 is equivalent to 100% efficiency (after n amplification cycles the number of molecules is 2^n). An equivalent set of serial dilutions gave efficiency for E1A amplification. The average efficiency of 1.945 for MVA and 1.975 for E1A was used to calculate number of molecules in the samples.

We have applied this rechnique also to an infection time course: CS and CS.pIX cells (as well as CR and CR.pIX cells, not shown) were infected with an MOI of 0.1. Cells were lysed by three cycles of freeze/thaw 15, 24, 48, 72 and 96 h post infection and subjected to titration for pfu/ml on Vero and viral DNA/ml by real time PCR. The ratio of viral genomic DNA/ml to pfu/ml is an estimate on the amount of DNA that is packaged into infectious virions. As can be seen in Fig. 14 there is an excess of viral DNA independent on the presence of pIX early in the infectious cycle (15 h to 24 h post infection). However, at late stages of the infectious cycle (48 h to 96 h) pIX-positive cells appear to require fewer copies of genomic viral DNA to release a greater number of infectious particles than parental cells. This result suggests that in the presence of pIX fewer faulty copies are generated for packaging into viral particles. In a nutshell it can be stated, that not only more viruses - or target proteins- are formed but also that the virions are more infective - or, with the aid of chaperons during the expression, a product with better specific activity.

As the viral machinery of genome replication is especially sensitive to inactivation (Munyon et al. 1970 in J Virol 5, 32-38) the presence of pIX provides an unexpected benefit by stabilizing this component. The polymerase complex is important within the host cell to provide genomic DNA for packaging but also within progeny virions to initiate the infectious cycle: the isolated genome of most viruses is not infectious and must be interpreted by the cognate viral polymerase. Thus this enzyme is also packaged into virus particles and delivered together with the genome upon penetration into the host.

For several viruses it is known from the literature that heat shock and especially Hsp90 contribute to stability of the replication machinery (Connor, et al. 2007 in Virology 362, 109-119). Our results demonstrate that Hsp90 also stabilizes MVA polymerase. Defective particles often are the result of deletions in the genome and incomplete packaging (Barrett et al. 1984 in J Gen Virol 65 ( Pt 12), 2265-2268; Garoff et al. 1998 in Microbiol Mol Biol Rev 62, 1171-1190; McLain et al. 1988 in J Gen Virol 69 ( Pt 6), 1415-1419). In industrial processes defective particles interfere with yields and increase the burden for the recipients as even empty virions sometimes are immunstimulatory. Also, for recombinant viral vectors genetic instability can be a severe problem that determines whether a vaccine strategy can be implemented successfully or not (for example, Wyatt et al. 2008 in Virology 372, 260-272). Deletion within recombinant viruses is mediated by the replication machinery and any mechanism that stabilizes this enzyme complex can have massive effects on vector quality.

### Example 10 Induction of heat shock in various cell lines

Induction of heat shock is associated with formation of stress granules (SG) (reviewed in Kedarsha and Anderson 2002 in Biochem. Soc. Transactions 30, 963-969). Formation of these cytoplasmatic speckles is rapid. pIX can be visualized in live cells by fusion to a GFP reporter protein. If an expression plasmid of such a fusion protein is transfected into the cells using effectene as described above, within 5 hours formation of bright cellular inclusions consistent with stress granules are visible in various cell lines (Fig. 8). This indicates that pIX (and fusion proteins thereof) can be used in cells of various species and tissue origin for induction of heat shock responsens.

Furthermore, we were able to demonstrate that pIX delays cytopathic effect from MVA also in a cell line from the Egyptian fruit bat (DSMZ #ACC2902) (see Fig. 9 ). R06E is the parental line, R06E.p9GFP stably expresses the pIX-GFP fusion protein. Note that at day 13 in the pIX-GFP-expressing line almost no cytopathic effect is visible, in the parental line fulminant infection clearly is apparent. After further passaging, at day 17, the pIX-expressing cell lines suffers strong cytopathic effect; the parallel culture with parental cells at that time was killed by the virus.

### Example 11 Non-toxic maintenance of heat shock

Heat shock proteins, especially Hsp90 and Hsp70, are proposed to buffer normal cells against environmental insults with the effect of suppressing mutations and increasing phenotypic stability (Nollen & Morimoto 2002 in Journal of Cell Science 115, 2809-2816; and Queitsch et al. 2002 in Nature 417, 618-624). Constitutive activation of heat shock therefore should be highly beneficial for a cell line, especially for a cell line to be used in demanding clinical environment and bioreactor applications.

However, most heat shock proteins already are constitutively expressed in normal cells (Nollen & Morimoto 2002 in Journal of Cell Science 115, 2809-2816) without concomitant activation of heat shock pathways. Thus to induce heat shock mere over-expression of certain heat shock proteins is not sufficient. To induce heat shock experimentally proteotoxic stressors such as isopropanol or CdCl are added. However, this induces heat shock as a response to damage and stress rather in a proactive fashion as is possible with the viral factor pIX that possibly functions in the adenovirus infectious cycle as a factor preparing the cell for the imminent burden of generating enormous amounts of progeny virus.

Heat shock proteins consume ATP for chaperone activity. Cells with constitutively activated heat shock are expected to shift in metabolic requirement. We observed changes in amino acid consumption and decreased proliferation rates in cells that stably express pIX protein. To recognize and adapt to the shifted requirements of the pIX-expressing cell lins appears to be one explanation instrumental for success in generating lines that stably express this factor. It is known from the literature that pIX is not expressed easily in cell lines:
For example, in Krougliak & Graham (1995 in Hum Gene Ther. 6, 1575-86) on page 1580 state that constituive expression of pIX (clone VK4-24) results in an unstable clone that looses pIX expression within 15 passages.

Imler et al. (1996 in Gene Ther. 3, 75-84) on pages 81 and 82 also discuss that pIX is not stably expressed in either A549 or 293 clones transfected for stable pIX expression.

In Caravokyri & Leppard (1995 in J. Virol. 69, 6627-6633) the authors on page 6629 state that pIX appears to interfere with episome maintenance and therefore use a 293 clone that stably expresses the cognate factor EBNA1 that is responsible for episome amplification. Furthermore they state that "some adverse effect of pIX expression on cell viability was observed during initial selection", and this effect correlated with strength of the promoter driving pIX expression.

Consequently, previously pIX expression was performed with conditional (or inducible) promoters or an episomal (non-integrated) expression system.

To analyze maintenance of PIX in the stably transfected cells genomic DNA was isolated from 1 x 10^6 cells with the QIAamp DNA Blood kit (Qiagen) and number of E1B and PIX molecules was determined in 25 ng and 50 ng of genomic DNA in an ABI 7000 TaqMan reaction with SYBR Green (ABI) detection chemistry according to the instructions by the manufacturer. Primers used for quantification were gTggTTgCTTCATgCTAgTg (SEQ ID NO: 14) and TCTTCAgCAggTgACAgTTg (SEQ ID NO:15) for E1B, ACCTACgAgACCgTgTCTg (SEQ ID NO:16) and gAgCCgTCAACTTgTCATC (SEQ ID NO:17) for pIX. Amplicon sizes are 123 bp and 166 bp for E1B and pIX, respectively. As E1B was introduced independently and must be maintained for the cells to survive this gene serves as an internal marker to standardize PIX copy number and gene expression strength.

Fig. 10 in panels (C) and (B) demonstrates stable maintenance of pIX transgene in CR suspension cells even in absence of selection pressure. Panel (A) demonstrates by RT-PCR that amount of mRNA expressed in CR.pIX cells is approximately 10-fold higher than amount of E1B mRNA. Panel (B) also demonstrates that pIX-positive cells suffer from a decrease in growth rate also if cells are allowed to proliferate without hygromycin in the medium. We consistently observed decreased proliferation for all of our cells (avian, human or bat cells) upon stable transfection with a pIX-expression plasmid demonstrating an impact of pIX on the biochemistry of the host cell. Panel (D) shows agarose gels of an RT-PCR with a different set of pIX primers (described

To confirm industrial usability we next examined whether a pIX-expressing cell line can proliferate in a stirred tank bioreactor at high cell viabilies and whether that culture can be infected with MVA at low MOI of 0.05 in a fed batch procedure to yield high yields. Fig. 11 demonstrates that this demanding procedures is possible with a pIX-positive cell line. A DASGIP bioreactor was inoculated with CR.pIX suspension cells in AEM (Invitrogen #12582-011) medium at cell density of 1 x 10^6/ml and the culture was allowed to proliferate with 50% oxygen saturation at segmented impeller speed of 150 rpm and final volume of 400 ml; pH was controlled for fixed 6.8 units. At a cell density above 4 x 10^6 cells/ml one volume of supplemented DMEM/F12 medium was added together with MVA at an MOI of 0.05. Samples were removed 24, 48 and 72 h post infection for titration of virus. MVA is a difficult virus to produce and yet titers beyond 5 x 10^7 easily were obtained in a generic DASGIP run without further optimization. The micrographs in Fig. 11 demonstrate a healthy and homogenous cell population prior to infection and rapid aggregate formation at infection.

### Example 12 Stabilization of cell phenotpye

Heat shock response is beneficial to the cell under conditions of high stress. It is therfore not surprising that a large body of literature (for example: Ait-Aissa et al. 2000 in Toxicology 145, 147-157; Neuhaus-Steinmetz & Rensing 1997 in Toxicology 123, 185-195; Meyer et al. 1995 in Appl. Environ. Microb. 61, 979-984; Rosner & Aumercier 1990 in Antim. Agents Chemother. 34, 2402-2406) indicates that experimental induction of heat shock often is by secondary pathways that first induce protein denaturation and cell cytotoxicity that subsequently is communicated into the heat shock response.

A specific (rather than a proteotoxic) inducer of heat shock is expected to stabilize cells. To demonstrate that pIX induces heat shock without rendering the cell more sensitive to environmental stress cells that stably express pIX were exposed to salicylate and indomethacin. Parental cells served as control.

Salicylate has a multitude of activities, some involved in genetic induction of heat shock (Jurivich et al. 1995 in J. Biol. Chem. 41, 24489-24495); this compound is toxic to some cells (Hughes et al. 2003 in Biochem. J. 374, 481-488). Indomethacin also is toxic to some cells. This compound destabilizes proteins within the cell and thus unspecifically induces heat shock (Roussou et al. 2000 in Cell Stress & Chaperones 5, 8-13). It can be seen in Fig. 12 that in presence of pIX cells were more resistant to salicylate (toxicity clearly visible only in the parental cell line at 2 mM) and more resistant to indomethacin (at 100 µM clear cytotoxicity visible in the parental line but not in the pIX derivative).

This protective effect extends to a product produced by the cell lines: the cells were infected with MVA at an MOI of 0.1 and treated with salicylate and indomethacin. Yields of MVA clearly are superior in the CS.pIX cells compared to parental CS cells. In Fig. 13 the second (right) ordinate depicts fold-increase. In spite of indomethacin treatment MVA yields are 5-fold higher in presence of pIX, in spite of salicylate treatment MVA yields are up to 20-fold higher in presence of pIX. This result with two independent compounds indicates that in presence of pIX production processes should occur with less sensitivity to detrimental environmental stimuli. Robust production processes at high yields are an important industrial determinant.

### Example 13 Screen for heat shock modulation

A number of infectious agents and massive proliferative turnover of tumor cells requires the chaperone activity of Hsp90. Hsp90 is involved in stabilization of viral replication complexes, and in maintaining conformation of factors involved in cell cycle progression such as AKT and cyclin-dependent kinase 4. Thus, small molecules interfering with activity of Hsp90 are promising compounds against certain viral infections and certain malignant neoplasm.

High-throughput screens for such compounds are instrumental for discovery and characterization of novel therapeutic candidates. Currently, Hsp90 inhibitors are screened for via enzymatic acitvity of Hsp90 requiring purification of this factor (Rowlands et al. 2004 in Analytical Biochemistry 327, 176-183). Hsp90 is active in aggregates. Compounds that interfere with formation of these aggregates leading towards activation of Hsp90 would not be indetified in such a narrow screen. Recently, a cell based assay was developed (Hardcastle et al. 2007 in Mol Cancer Ther 6, 1112-22). However, this assay also requires substantial down-stream manipulation. The read-out is increase in Hsp70 expression.

Using two cell lines, a cell stably expressing pIX and a parental cell line, one can screen compounds with very little downstream processing. As indicated in Fig. 15 suitable inhibitors of heat shock response can be identified by differential cytotoxicity of compounds to be screened for the two cell lines. Screening for cytotoxicity can be performed in convenient MTT staining of mitochondria in life cells. Such methods are well known in the art. The main hurdle for establishment of such a robust assay of low complexitiy is providing a cell line with constitutively activated heat shock and having a truly parental cell line as reference. Induction of heat shock by chemical inducers such as CdCl, isopropanol and acrylamide would interfere with the measurement of cytotoxicity.

The specificity of the assay can also be ssen in Fig. 15 which we tested with PDTC. PDTC is an inhibitor of NF-kB activity (Gaudernak et al. 2002 in J Virol 76, 6004-6015). NF-kB is an important sensor molecule and transcription activator mediating inflammation. Thus, NF-kB also can be involved in stress response pathways and sometimes is induced or inhibited by viruses depending on requirement of the parasite (Santoro et al. 2003 in EMBO J 22, 2552-2560). However, in the pIX-expressing cells this factor appears not to be influenced and consequently molecules interfering with this pathways are not differentially activated in the two cell lines. PDTC does interfere with MVA replication but presence or absence of pIX has no effect on the extend of this inhibition.

## Claims

1. Method for preparing a cell having stably integrated into its genome a gene coding for a heterologous regulator protein or a functional variant thereof and stably expressing said regulator protein or the functional variant thereof and stably expressing said regulator protein, **characterized in that** the cell is capable of permanently sustaining a heat shock cascade without going into apoptosis.

2. A method according to claim 1, wherein the cell is obtained by transfecting the cell with a vector carrying said regulator protein or the functional variant thereof.

3. Method according to claim 1 or 2, wherein the cell is infected with a virus, or transfected with a vector carrying a nucleic acid sequence encoding a *virus* or said one or more target proteins.

4. Method according to any of the claims 1 to 3, wherein the heterologous regulator protein is pIX.

5. Method according to any of the claims 1 to 4, wherein the cell is an avian cell.

6. Method according to any of the claims 1 to 5, wherein the cell and the products of the protein expression are stabilized by a heat shock cascade.

7. Method according to any of the claims 1 to 6, wherein the heat shock cascade is activated selectively by HSP90.

8. Method according to any of the claims 1 to 7, wherein the products of the protein expression are a virus or one or more target proteins.

9. Method according to any of the claims 1 to 8 further comprising the step of preparing a target virus or one or more target proteins and isolating the virus or the target protein(s).

10. Method according to any of claims 5 to 9, wherein the host and expression cell is an avian cell, preferably derived from duck retina or duck somite, and said cell carries a nucleic acid sequence encoding Adenovirus PIX or a functional variant thereof as a heterologous regulator protein.

11. Method according to any of claims 1 to 4 and 6 to 9, wherein the host cell is a NC5T11#34 cell and the expression cell is derived from the NC5T11#34 cell, said NC5T11#34 cell being deposited with the DSMZ under accession number DSM ACC2744.

12. The method of claim 10, wherein the host cell is a CR.PIX (17a11b) cell and the expression cell is derived from the CR.PIX (17a11b) cell, said CR.PIX (17a11b) cell being deposited with the DSMZ under accession number DSM ACC2749.

13. Use of the expression cell obtained by a method according to any of the claims 1 to 11 for preparing a target virus or one or more target proteins.

14. Use according to claim 13, **characterized in that** the target virus or one or more target proteins are obtained in a higher ratio per time.

15. Use according to claim 13, **characterized in that** the target virus or one or more target proteins are obtained in a higher specific activity.

16. Use of a cell obtained by a method according to any of the claims 1 to 11 in a high throughput assay for inhibitors of the heat shock cascades.

17. Use according to claim 16, **characterized in that** the inhibitors inhibit HSP90.
